Europäisches Patentamt

(19) European Patent Office

Office européen des brevets

(11) Publication number: **0 083 969**
A2

# EUROPEAN PATENT APPLICATION

(21) Application number: 83300047.4

(22) Date of filing: 05.01.83

(51) Int. Cl.³: **G 01 N 27/48**
G 01 N 33/00, G 01 N 33/48
A 61 B 5/02

(30) Priority: 06.01.82 GB 8200328

(43) Date of publication of application:
20.07.83 Bulletin 83/29

(84) Designated Contracting States:
DE FR GB IT NL SE

(71) Applicant: CRITIKON, INC.
1410 N. Westshore Boulevard
Tampa Florida 33607(US)

(72) Inventor: Parker, Dawood
33 Woodland Gardens
London N10 3UE(GB)

(74) Representative: Colgan, Stephen James et al,
CARPMAELS & RANSFORD 43 Bloomsbury Square
London WC1A 2RA.(GB)

(54) Polarographic gas sensors.

(57) Thin film polarographic gas sensors are described wherein the electrodes are deposited on a flexible substrate. A bilumen monopolar catheter-type sensor has a thin cathode layer (8) deposited on the wall of one lumen (4), the distal end of which is sealed by a polyurethane plug (10) such that only the outer edge surface of the cathode layer (8) is in contact with a dip-coated permeable membrane (14). The cathode layer (8) extends to the proximal end of the catheter (2) where electrical contact is made with a conductor wire (16) through electrically-conductive epoxy resin (18). When the sensor is employed umbilically or intravascularly, the second lumen (6) may be employed to withdraw samples from the patient or supply fluids thereto.

The cathode layer (8) may be deposited on the catheter material in long lengths, which are then subsequently cut to the appropriate length for individual catheter-type sensors.

FIG. 2

EP 0 083 969 A2

## "Polarographic Gas Sensors"

### Field of the Invention

This invention relates to polarographic gas sensors and particularly to the manufacture of such sensors for use in blood gas monitoring.

### Background of the Invention

Polarographic sensors are an established approach to the measurement of gas concentration. They are used particularly for the sensing of oxygen partial pressures in blood, or for monitoring of constituent gases applied to a patient during anaesthesia.

Generally these sensors comprise at least one electrode separated from the medium under investigation by a permeable membrane. If a single electrode is employed, it is used as a cathode in conjunction with a remote reference electrode (anode), and is termed a monopolar sensor. The sensor can incorporate both anode and cathode, in which case it is termed a bipolar electrode. A DC potential is set up between the electrodes to cause polarisation of the cathode. Gas diffuses through the permeable membrane and, provided the potential is set at the correct level, the electrochemical reaction of the gas depolarises the electrodes and the consequent current flow can be measured as a representation of gas concentration in the medium.

Sometimes a small quantity of electrolyte (often gelled) is employed in the sensor, enclosed by the permeable membrane, but with certain hydrophilic membranes this is unnecessary, as the membrane itself can incorporate the electrolyte.

An early description of such a sensor is given in U.S. Patent 2,913,386 (L.C. Clark). More modern polarographic sensors are described for example in U.S. Patent 3,826,730 (Watanabe et al) and in European Patent Specification 0015075 (McNeilab Inc.).

The latter document describes a catheter-type polarographic sensor for umbilical or intravascular use, particularly in neonatals. The catheter has a bilumen construction: one lumen incorporating the polarographic sensor (cathode wire, gelled electrolyte, membrane) whereas the other lumen is left open to act as a channel enabling fluid samples to be taken from the patient or fluids to be supplied thereto.

The construction of such sensors needs to be accurately controlled to enable repeatable results to be obtained and the positioning and dimensions of the cathode are critical. There is a need for a simple method of manufacturing catheter-type sensors to enable large quantities to be manufactured easily and accurately.

Summary of the Invention

In accordance with a first aspect of the invention there is provided a polarographic gas sensor comprising a flexible elongate substrate onto the distal end of which is deposited a layer of an electrode material as an annular ring, and an electrically-conductive material in electrical contact with said electrode material and extending to the proximal end of said substrate.

The electrically-conductive material may be a conductive wire extending the length of the elongate substrate, but preferably it is the same material as the electrode material and exists as a layer deposited on the substrate. In the latter case, the layer of electrode material extends from the distal to the proximal end of the substrate and is present, at least at the distal end, as the annular ring.

The outer edge of the annular ring is exposed to enable the electrode material to act as the polarographic cathode. The outer edge is optionally in contact with and overlaid by a permeable membrane, but this is not essential as membrane-less polarographic gas sensors are known in the art. A membrane is preferred as this isolates the cathode from the fluid under investigation. However, isolation may also be achieved by, for example, maintaining the outer edge of the annular ring in a narrow recess so that relatively large fluid particles (such as blood particles) cannot make contact with the cathode.

In accordance with a second aspect of the invention there is provided a method of manufacturing a polarographic gas sensor which comprises providing a flexible elongate substrate material, depositing a layer of an electrode material onto the substrate material, with the deposited layer extending from the distal to the proximal end of the length of substrate material with an annular ring of electrode material existing at the distal end, and optionally coating the distal end with a permeable membrane such that the outer edge of said annular ring is in contact with and overlaid by said membrane.

Description of the Drawings

Figure 1 shows schematically steps in the process of manufacturing a monopolar catheter-type polarographic sensor for in vivo $pO_2$ measurement in blood, according to a first embodiment of the invention;

Figure 2 is a cross-section of the finished sensor of Figure 1;

Figure 3 shows schematically steps in the process of manufacturing a bipolar catheter-type polarographic sensor for _in vivo_ $pO_2$ measurement in blood, according to a second embodiment of the invention;

Figure 4 is a cross-section of the finished sensor of Figure 3; and

Figure 5 is a cross-section through a monopolar polarographic sensor for _in vivo_ $pO_2$ measurement in blood, according to a third embodiment of the invention.

Description of the Preferred Embodiments

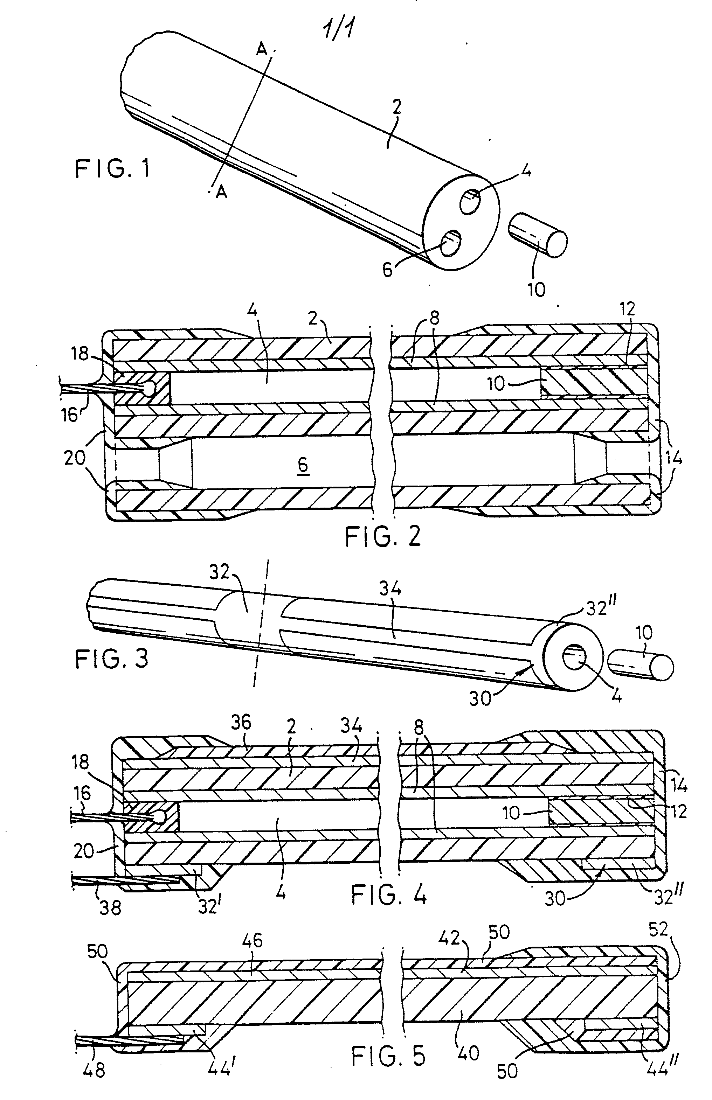

Referring to Figures 1 and 2 there is illustrated a monopolar bilumen catheter-type polarographic sensor and its manufacture.

Initially, a flexible catheter 2 of a synthetic plastics material such as polyurethane and having lumens 4 and 6 is provided. The wall of lumen 4 is coated with a thin layer 8, about 5 μm thick, of a noble metal such as platinum or gold. This layer 8 acts as the cathode in the sensor. The layer 8 may be applied by any of several techniques – by injecting a noble metal-containing paint into lumen 4, or by electrochemical deposition – electroplating or electroless plating. It may be feasible to vacuum deposit the metal on the wall of lumen 4. The layer 8 extends from the proximal to the distal end of catheter 2, thus providing electrical connection between these ends.

A polyurethane plug 10 having its curved surface coated with curable epoxy resin 12 is inserted into the distal end of lumen 4 and the resin allowed to set. The end of the catheter and plug are trimmed so that the outer end surface of plug 10 is flush with the distal end surface of catheter 2. In this manner only the end surface of layer 8 is exposed.

The distal end is then provided with a hydrophilic permeable membrane 14, for example, of polyhema, by dipcoating techniques as are well known in the art. A slight vacuum is applied to lumen 6 to cause the forming membrane to collapse therein and thus not obstruct the opening at the distal end of lumen 6.

A conductor wire 16 is inserted into the proximal end of lumen 4 and permanently fixed in electrical contact with layer 8 by means of electrically-conductive epoxy resin 18. An electrically-insulating varnish layer 20 is finally applied to the proximal end to isolate the electrical connection between conductor wire 16 and layer 8.

The monopolar sensor thus constructed may be employed for _in vivo_ $pO_2$ measurement in, for example, neonatals, in conjunction with a remote anode fixed transcutaneously to the patient in the normal manner. The second lumen 6 may be employed for body fluid sampling or for supplying fluid to the patient, and the proximal end of lumen 6 is attached to known types of interconnectors used for such purposes.

Because the cathode 8 is applied as a surface coating as opposed to being supplied as a discrete wire extending within lumen 4 (and which requires careful positioning therein), it is possible to use this technique for easily manufacturing sensors in large quantities. In such a circumstance a long length of the catheter material is first provided with the coating layer 8 prior to being cut into shorter lengths (e.g. at A-A in Figure 1) for individual processing as described.

Referring to Figures 3 and 4, there is shown a bipolar catheter-type polarographic sensor which omits the second lumen - for body fluid sampling or fluid supply.

The catheter is manufactured similarly to that of the previous embodiment and common features have been given identical reference numerals. In addition, however, the initial length of catheter material has applied to its exterior surface an electrically-conductive layer 30 of a noble metal such as silver, formed in the same manner as layer 8. The layer 30 is formed as a repeating pattern of bands 32 interconnected by strips 34. The repeat frequency of bands 32 is arranged so that the bands coincide with the cutting positions A-A from which the sensor is formed. In this manner both the proximal and distal ends of each cut length are provided with half width bands 32' and 32". Layer 30 acts as the anode in the sensor.

Prior to dipcoating so as to provide the permeable membrane 14, the strip 34 is coated with an electrically-insulating varnish 36. A conductor wire 38 is attached to the proximal band 32', for example with electrically-conducting epoxy resin and the whole of the proximal end coated with the electrically-insulating varnish 20.

It is not necessary that the cathode layer 8 be applied to the wall of lumen 4 - it may be applied to the exterior surface of catheter 2. Indeed, if a catheter is not required, a monopolar sensor may be constructed as shown in Figure 5.

Referring to Figure 5, the flexible catheter 2 is replaced by a continuous flexible polyurethane filament 40. Filament 40 is provided with a noble metal surface coating 42 formed of bands 44', 44" and strip 46. The coating 42 is provided in the same manner as the anode coating 30 of the Figures 3, 4 embodiment. An electrical conductor 48 is attached to proximal band 44' and the proximal end, strip 46, and band 44" coated with an electrically-insulating varnish layer 50. The distal end is trimmed to expose through the varnish layer 50 just the outer edge surface of band 44". The distal end is then provided with a hydrophilic permeable membrane 52 as in the previous embodiments.

In a variation of the embodiment illustrated in Figures 1 and 2, the catheter can be adapted for bipolar use by filling lumen 6 with an electrically-conductive filler, for example silver-containing epoxy resin. Alternatively, if lumen 6 remains open and the catheter employed _in vivo_, it may be filled with saline and electrical contact made by inserting a reference electrode into the proximal end of lumen 6.

Although the invention has been especially described in relation to sensors for $pO_2$ monitoring, it will be appreciated that it is not restricted to such use and sensors manufactured according to the invention can be employed for monitoring of other gases.

Claims:-

1. A polarographic gas sensor comprising a flexible elongate substrate onto the distal end of which is deposited a layer of an electrode material as an annular ring, and an electrically-conductive material in electrical contact with said electrode material and extending to the proximal end of said substrate.

2. A sensor according to claim 1 wherein the electrode material extends from the distal to the proximal end and forms the electrically-conductive material.

3. A sensor according to claim 1 or 2 wherein the outer edge of the annular ring of electrode material is in contact with and overlaid by a permeable membrane.

4. A sensor according to any of claims 1 to 3 wherein the substrate comprises a flexible catheter.

5. A sensor according to claim 4 wherein the catheter comprises two lumens.

6. A sensor according to claim 4 or 5 wherein the layer is deposited on the wall of the, or one of the lumens of the catheter.

7. A sensor according to claim 6 wherein the distal end of the lumen bearing the layer of electrode material is closed with an electrically-insulating material so as to expose only the outer edge of said annular ring to said membrane.

8. A sensor according to any of claims 4 to 7 wherein first and second layers of electrode material are provided on the catheter extending from the distal to the proximal end, one layer on the wall of the, or one of the lumens of the catheter and the other on the outer surface of the catheter and providing concentric annular rings of electrode material at the distal end , the outer edges of which rings are in contact with and overlaid by said permeable membrane.

9. A sensor according to any of claims 1 to 8 wherein the substrate is a synthetic plastics material.

10.    A method of manufacturing a polarographic gas sensor which comprises providing a flexible elongate substrate material, depositing a layer of an electrode material onto the substrate material with the deposited layer extending from the distal to the proximal end of the length of substrate material, with an annular ring of electrode material existing at the distal end, and optionally coating the distal end with a permeable membrane such that the outer edge of said annular ring is in contact with and overlaid by said membrane.

11.    A method according to claim 10 which comprises depositing said layer onto a length of said substrate material and severing the latter into shorter lengths such that each cut length is employed to form an individual sensor.

12.    A method according to claim 11 or 12 wherein the layer is provided by depositing an electrically-conductive paint, by electrochemical deposition, or by vacuum deposition.

13.    A method according to any of claims 10 to 12 wherein the substrate material is a catheter.

14.    A method according to claim 13 wherein the layer is deposited on the wall of the lumen of the catheter.

15.    A method according to claim 14 wherein a separate layer of electrode material, extending from the distal to the proximal end, is provided on the outer surface of the catheter and providing, with the first annular ring, concentric annular rings of electrode materials whose outer edges are in contact with and overlaid by said permeable membrane.

16.    A method according to claim 14 or 15 wherein the distal end of said catheter is closed with an electrically-insulating material.

17. A polarographic gas sensor substantially as herein described with reference to Figures 2, 4 or 5 of the accompanying drawings.

18. A method of manufacturing a polarographic gas sensor substantially as herein described with reference to Figures 1 and 2, 3 and 4 or 5 of the accompanying drawings.

FIG. 1

FIG. 2

FIG. 3

FIG. 4

FIG. 5